# EUROPÄISCHE PATENTANMELDUNG

(11) **EP 1 270 731 A1**
(43) Veröffentlichungstag der Anmeldung: **02.01.2003**
(21) Anmeldenummer: 01114661.0
(22) Anmeldetag: 19.06.2001
(51) Int. Cl.: C12N 15/82, C12N 15/53, C12N 15/11, C12N 9/02, C07K 14/415, A01H 5/00, A01H 5/10, A23L 1/30

(54) **Verfahren zur Erhöhung des Carotinoid-Gehalts in transgenen Pflanzen**

(71) Anmelder: Dr. Kartz von Kameke, 24340 Friedensthal (DE)
(72) Erfinder: Dr. Kartz von Kameke, 24340 Friedensthal (DE)
(74) Vertreter: Maiwald Patentanwalts GmbH

(57) **Zusammenfassung**

Die Erfindung betrifft ein Verfahren zur Erhöhung des Carotinoid-Gehalts in transgenen Pflanzenzellen und Pflanzen. Insbesondere betrifft die Erfindung ein Verfahren zur Erhöhung des Carotinoid-Gehalts in transgenen Kartoffelzellen und -pflanzen. Des weiteren betrifft die Erfindung rekombinante Nukleinsäuremoleküle, die eine DNA-Sequenz enthalten, die ein Protein mit der enzymatischen Aktivität einer Zeaxanthin-Epoxidase (Zep) kodiert, und worin diese DNA-Sequenz unter Kontrolle von regulatorischen Sequenzen eines in Pflanzen aktiven Promotors, insbesondere eines Samen-, Frucht- und/oder Speicherorgan-spezifischen Promotors, steht.

SEQUENZPROTOKOLL

<110> Dr. von Kameke, Kartz

<120> Verfahren zur Erhöhung des Carotinoid-Gehalts in
      transgenen Pflanzen

<130> S 7475

<140>
<141>

<160> 2

<170> PatentIn Ver. 2.1

<210> 1
<211> 1044
<212> DNA
<213> Solanum tuberosum

<220>
<221> CDS
<222> (1)..(1044)

<400> 1
tgg tac tgt aag ttt gac acg ttc act cca gca gtg gaa cgt gga ctt    48
Trp Tyr Cys Lys Phe Asp Thr Phe Thr Pro Ala Val Glu Arg Gly Leu
  1               5                  10                  15

ccc gtg gca aga gtc atc agc cgc atg act ttg cag cag atc ctt gca    96
Pro Val Ala Arg Val Ile Ser Arg Met Thr Leu Gln Gln Ile Leu Ala
             20                  25                  30

cgt gct gtt ggg gag gat aca att atg aat gaa agt aat gtt gta gac    144
Arg Ala Val Gly Glu Asp Thr Ile Met Asn Glu Ser Asn Val Val Asp
         35                  40                  45

ttt gag gat gat ggg gag aag gtt agt gtg gtt att gag aat gga caa    192
Phe Glu Asp Asp Gly Glu Lys Val Ser Val Val Ile Glu Asn Gly Gln
     50                  55                  60

cga ttt aca ggt gat ctt ctg gtt ggt gct gat ggc ata agg tct aag    240
Arg Phe Thr Gly Asp Leu Leu Val Gly Ala Asp Gly Ile Arg Ser Lys
 65                  70                  75                  80

gta cgg act aat ttg ttt gga ccc agt gaa gtt act tac tct ggc tac    288
Val Arg Thr Asn Leu Phe Gly Pro Ser Glu Val Thr Tyr Ser Gly Tyr
                 85                  90                  95

act tgt tat act gga att gca gat ttt gtt cct gct gat att gat aga    336
Thr Cys Tyr Thr Gly Ile Ala Asp Phe Val Pro Ala Asp Ile Asp Arg
            100                 105                 110

gtt ggg tac cga gtc ttt ttg ggc cac aaa cag tac ttt gtt tct tca    384
Val Gly Tyr Arg Val Phe Leu Gly His Lys Gln Tyr Phe Val Ser Ser
        115                 120                 125

gat gtg ggg gga ggc aag atg cag tgg tat gca ttt tac aat gaa cca    432
Asp Val Gly Gly Gly Lys Met Gln Trp Tyr Ala Phe Tyr Asn Glu Pro
    130                 135                 140

gct ggt ggt gtg gat gct cca aat ggt aaa aag gaa aga ttg ctt aaa    480

Ala Gly Gly Val Asp Ala Pro Asn Gly Lys Lys Glu Arg Leu Leu Lys
145                 150                 155                 160

ata ttt ggg gga tgg tgt gac aat gtc ata gac cta tta gtt gcc aca    528
Ile Phe Gly Gly Trp Cys Asp Asn Val Ile Asp Leu Leu Val Ala Thr
                165                 170                 175

gat gaa gat gca att ctt cgt cgt gac atc tat gat aga cca cca act    576
Asp Glu Asp Ala Ile Leu Arg Arg Asp Ile Tyr Asp Arg Pro Pro Thr
            180                 185                 190

ttt agt tgg gga aga ggt cat gtt aca ttg ctt ggg gac tct gtc cat    624
Phe Ser Trp Gly Arg Gly His Val Thr Leu Leu Gly Asp Ser Val His
        195                 200                 205

gct atg cag cct aat ttg ggt caa ggg gga tgc atg gcc ata gag gat    672
Ala Met Gln Pro Asn Leu Gly Gln Gly Gly Cys Met Ala Ile Glu Asp
    210                 215                 220

agc tat caa cta gca ctg gaa ctt gac aaa gca tgt agt cga agt gcc    720
Ser Tyr Gln Leu Ala Leu Glu Leu Asp Lys Ala Cys Ser Arg Ser Ala
225                 230                 235                 240

gag tca gga agc cct gtg gat atc atc tca tct tta agg agc tat gaa    768
Glu Ser Gly Ser Pro Val Asp Ile Ile Ser Ser Leu Arg Ser Tyr Glu
                245                 250                 255

agt gct aga aaa ctt cga gtt gga gtc atc cat gga ctg gct aga atg    816
Ser Ala Arg Lys Leu Arg Val Gly Val Ile His Gly Leu Ala Arg Met
            260                 265                 270

gct gca atc atg gca tct act tac aaa gct tat ctt ggc gtc gga ctt    864
Ala Ala Ile Met Ala Ser Thr Tyr Lys Ala Tyr Leu Gly Val Gly Leu
        275                 280                 285

ggt cca cta tca ttt ttg acg cag tat aga ata cca cat cct gga aga    912
Gly Pro Leu Ser Phe Leu Thr Gln Tyr Arg Ile Pro His Pro Gly Arg
    290                 295                 300

gtt ggt gga aga gta ttt att gac ttg gga atg cct ctg atg tta agt    960
Val Gly Gly Arg Val Phe Ile Asp Leu Gly Met Pro Leu Met Leu Ser
305                 310                 315                 320

tgg gtt cta gga ggc aat ggg gac aag ctt gaa ggc aga ata aaa cat    1008
Trp Val Leu Gly Gly Asn Gly Asp Lys Leu Glu Gly Arg Ile Lys His
                325                 330                 335

tgc agg cta tct gag aaa gca aat gac caa ttg aga                    1044
Cys Arg Leu Ser Glu Lys Ala Asn Asp Gln Leu Arg
            340                 345

<210> 2
<211> 348
<212> PRT
<213> Solanum tuberosum

<400> 2
Trp Tyr Cys Lys Phe Asp Thr Phe Thr Pro Ala Val Glu Arg Gly Leu
1               5                   10                  15

Pro Val Ala Arg Val Ile Ser Arg Met Thr Leu Gln Gln Ile Leu Ala
            20                  25                  30

Arg Ala Val Gly Glu Asp Thr Ile Met Asn Glu Ser Asn Val Val Asp
        35                  40                  45

Phe Glu Asp Asp Gly Glu Lys Val Ser Val Val Ile Glu Asn Gly Gln
    50                  55                  60

Arg Phe Thr Gly Asp Leu Leu Val Gly Ala Asp Gly Ile Arg Ser Lys
65                  70                  75                  80

Val Arg Thr Asn Leu Phe Gly Pro Ser Glu Val Thr Tyr Ser Gly Tyr
                85                  90                  95

Thr Cys Tyr Thr Gly Ile Ala Asp Phe Val Pro Ala Asp Ile Asp Arg
            100                 105                 110

Val Gly Tyr Arg Val Phe Leu Gly His Lys Gln Tyr Phe Val Ser Ser
        115                 120                 125

Asp Val Gly Gly Gly Lys Met Gln Trp Tyr Ala Phe Tyr Asn Glu Pro
    130                 135                 140

Ala Gly Gly Val Asp Ala Pro Asn Gly Lys Lys Glu Arg Leu Leu Lys
145                 150                 155                 160

Ile Phe Gly Gly Trp Cys Asp Asn Val Ile Asp Leu Leu Val Ala Thr
                165                 170                 175

Asp Glu Asp Ala Ile Leu Arg Arg Asp Ile Tyr Asp Arg Pro Pro Thr
            180                 185                 190

Phe Ser Trp Gly Arg Gly His Val Thr Leu Leu Gly Asp Ser Val His
        195                 200                 205

Ala Met Gln Pro Asn Leu Gly Gln Gly Gly Cys Met Ala Ile Glu Asp
    210                 215                 220

Ser Tyr Gln Leu Ala Leu Glu Leu Asp Lys Ala Cys Ser Arg Ser Ala
225                 230                 235                 240

Glu Ser Gly Ser Pro Val Asp Ile Ile Ser Ser Leu Arg Ser Tyr Glu
                245                 250                 255

Ser Ala Arg Lys Leu Arg Val Gly Val Ile His Gly Leu Ala Arg Met
            260                 265                 270

Ala Ala Ile Met Ala Ser Thr Tyr Lys Ala Tyr Leu Gly Val Gly Leu
        275                 280                 285

Gly Pro Leu Ser Phe Leu Thr Gln Tyr Arg Ile Pro His Pro Gly Arg
    290                 295                 300

Val Gly Gly Arg Val Phe Ile Asp Leu Gly Met Pro Leu Met Leu Ser
305                 310                 315                 320

Trp Val Leu Gly Gly Asn Gly Asp Lys Leu Glu Gly Arg Ile Lys His
                325                 330                 335

Cys Arg Leu Ser Glu Lys Ala Asn Asp Gln Leu Arg
            340                 345

## Beschreibung

Die Erfindung betrifft ein Verfahren zur Erhöhung des Carotinoid-Gehalts in transgenen Pflanzenzellen und Pflanzen. Insbesondere betrifft die Erfindung ein Verfahren zur Erhöhung des Carotinoid-Gehalts in transgenen Kartoffelzellen und -pflanzen. Des weiteren betrifft die Erfindung rekombinante Nukleinsäuremoleküle, die eine DNA-Sequenz enthalten, die ein Protein mit der enzymatischen Aktivität einer Zeaxanthin-Epoxidase (Zep) kodiert, und worin diese DNA-Sequenz unter Kontrolle von regulatorischen Sequenzen eines in Pflanzen aktiven Promotors, insbesondere eines Samen-, Frucht- und/oder Speicherorgan-spezifischen Promotors, steht.

Weiter betrifft die Erfindung Vektoren und Wirtszellen, die die erfindungsgemäßen rekombinanten Nukleinsäuremoleküle enthalten. Die für eine Zeaxanthin-Epoxidase kodierende DNA-Sequenz liegt in den erfindungsgemäßen Nukleinsäuremolekülen in Senseoder Antisense-Orientierung vor, und die Übertragung der Nukleinsäuremoleküle auf Pflanzenzellen führt zu einer Co-Suppression respektive Antisense-Inhibierung der endogenen Carotinoid-Epoxidation in den transgenen Pflanzenzellen.

Ferner betrifft die Erfindung transgene Pflanzen und Pflanzenzellen, die ein erfindungsgemäßes Nukleinsäuremolekül enthalten und aufgrund dessen im Vergleich zu Wildtyp-Pflanzen bzw. -Zellen einen erhöhten Gehalt an Zeaxanthin und/oder einen erhöhten Gehalt an Gesamtcarotinoid aufweisen, sowie Ernteprodukte und Vermehrungsmaterial der transgenen Pflanzen.

Die Erfindung betrifft des weiteren ein Verfahren zur Herstellung von Zeaxanthin und anderen Carotinoiden in Algen.

Schließlich betrifft die Erfindung die Verwendung von DNA-Sequenzen, die für Proteine mit der enzymatischen Aktivität einer Zeaxanthin-Epoxidase kodieren, zur Erhöhung des Zeaxanthin- und/oder Gesamt-Carotinoid-Gehalts in transgenen Pflanzen.

Bei den Carotinoiden handelt es sich um gelbe bis rote Pigmente, die in Mikroorganismen und Pflanzen synthetisiert werden. In Pflanzen akkumulieren Carotinoide in den Chloroplasten sowie in Chromoplasten von Blüten, Früchten oder anderem Gewebe. Die typischen zyklischen Pflanzencarotinoide enthalten ein konjugiertes Doppelbindungssystem und Ionon-Endgruppen, die durch Keto-, Hydroxy- und Epoxygruppen in verschiedenen Positionen substituiert sein können (Sandmann (2001) Arch. Biochem. Biophys. 385, 4-12). Die Carotinoide zeichnen sich besonders durch ihre äußerst wichtige Funktion als lipophile Antioxidantien aus, dank derer sie in der Lage sind, Zellen gegen oxidative Schädigung, insbesondere der Membranen und anderer lipophiler Komponenten zu schützen. Aus dieser wichtigen Rolle der Carotinoide als Antioxidantien leiten sich auch ihre nützlichen Wirkungen für die Gesundheit von Mensch und Tier ab.

So wird gegenwärtig angenommen, daß Carotinoide aufgrund ihrer antioxidativen Eigenschaften helfen können, degenerativen Erkrankungen vorzubeugen. Eine weitere wichtige Funktion von Carotinoiden mit mindestens einer nicht-substituierten β-Ionon-Gruppe besteht in der Bereitstellung von Provitamin A.

Lutein und Zeaxanthin sind die Hauptcarotinoide in der menschlichen Netzhaut (Landrum & Bone (2001), Arch. Biochem. Biophys. 385, 28-40). Auch hier schützen diese Carotinoide die Netzhaut vor Schädigungen durch Licht und höchst wahrscheinlich auch gegen Singulett-Sauerstoff. Altersbedingte Makulardegeneration ist die Hauptursache für Erblindung. Das Risiko der altersbedingten Makulardegeneration und damit der hierauf zurückzuführenden Erblindung kann durch gesteigerte Aufnahme von Zeaxanthin und Lutein mit der Nahrung bzw. als Nahrungsergänzung verringert werden (Seddon et al. (1994) J. Am. Diet. Assoc. 273, 1413-1420). Während Lutein in Gemüse in ausreichenden Mengen vorhanden ist, sind die Zeaxanthin-Gehalte in unserer Ernährung sehr gering (van den Berg et al. (2000) J. Sci. Food Agric. 80, 880-912). Zu den wenigen natürlichen Quellen von Zeaxanthin ((3R,3'R)-β,β-Carotin-3,3'-diol) zählen Mikroorganismen und spezielle gelbe/orangefarbene Paprikasorten (Minguez-Mosquera & Hornero-Mendez (1994) J. Agric. Food. Chem., 42, 1555-1560). Des weiteren findet man Zeaxanthin in Mais, aus dem es 1939 erstmals isoliert wurde. Von Zea mays leitet sich auch der Name Zeaxanthin ab.

Im Stand der Technik wurde bereits versucht, den Gehalt an Carotinoiden in Pflanzen mittels gentechnischer Modifikation des Carotinoid-Biosyntheseweges zu erhöhen. So konnte beispielsweise der Gehalt von β-Carotin mit Provitamin A-Aktivität durch Übertragung von Genen des Carotinoid-Stoffwechsels gesteigert werden. In Tomatenfrüchten war die Synthese zugunsten von β-Carotin verschoben (Römer et al. (2000) Nature Biotechnol. 18, 666-669; Rosati et al. (2000), Plant J. 24, 413-419), und in Rapssamen konnte der β-Carotin-Gehalt um das fünfzigfache erhöht werden (Shewmaker et al. (1999) Plant J., 20, 401-412). Die β-Carotin-Synthese konnte sogar in Gewebe bewirkt werden, das normalerweise keine Carotinoid-Biosynthese aufweist. So konnte durch die Übertragung mehrerer Gene, die für Enzyme kodieren, welche die einleitenden Schritte des Carotinoid-Stoffwechsels katalysieren, β-Carotin-Synthese in Reissamen erzielt werden (Ye et al. (2000) Science 287, 303-305).

In WO 99/55889 werden für Zeaxanthin-Epoxidase kodierende DNA-Sequenzen aus Mais und Soja beansprucht, allerdings ohne jede experimentelle Evidenz dafür zu liefern, daß es sich bei den offenbarten Sequenzen auch tatsächlich um Zep-Sequenzen handelt. Transgene Pflanzen mit erhöhtem Zeaxanthin-Gehalt werden in WO 99/55889 ebenso wenig offenbart wie Pflanzen mit einem erhöhten Gehalt an Gesamt-Carotinoiden.

Eine erfolgreiche Steigerung des Zeaxanthin-Gehalts in transgenen Pflanzen ist bislang nicht beschrieben worden. Aber gerade der Zeaxanthin-Gehalt in Nutzpflanzen ist für die Landwirtschaft und die Nahrungsmittelindustrie von größtem Interesse.

Eine Aufgabe der vorliegenden Erfindung ist somit die Bereitstellung von transgenen Pflanzen, die einen im Vergleich mit ihrer Wildtyp-Form erhöhten Gehalt an Zeaxanthin aufweisen und damit als Nahrungsmittel höherwertiger sind.

Eine weitere Aufgabe der vorliegenden Erfindung liegt in der Bereitstellung von DNA-Sequenzen, durch deren Übertragung ein erhöhter Gehalt an Zeaxanthin in transgenen Nutzpflanzen, insbesondere in Kartoffel, erzielt werden kann.

Da der Gesamtgehalt an Carotinoiden in Nutzpflanzen den Wert der Pflanze bzw. ihrer Organe für die menschliche und tierische Ernährung ebenfalls bestimmt, besteht eine weitere Aufgabe der Erfindung darin, den Gesamtgehalt an Carotinoiden in transgenen Nutzpflanzen zu erhöhen.

Es ist eine weitere Aufgabe der Erfindung, ein Verfahren zur Erhöhung des Gehalts an Zeaxanthin bzw. ein Verfahren zur Produktion von Zeaxanthin in anderen Wirtszellen bzw. Organismen, die zur Herstellung von Zeaxanthin natürlicherweise befähigt sind, bereitzustellen. Als Wirtszellen/-organismen sind hier vor allem Algen, insbesondere Grünalgen und höhere Algen, und pflanzliche Zellkulturen, wie Kalluskulturen, Suspensionskulturen, zu nennen. Das Zeaxanthin wird aus den Wirtszellen/-organismen bzw. Zellkulturen mit gesteigerter Zeaxanthinproduktion gewonnen und kann der Nahrung von Mensch und Tier zugesetzt werden, z.B. als Nahrungsergänzung.

Weitere Aufgaben der Erfindung ergeben sich aus der nachfolgenden Beschreibung. Die der Erfindung zugrundeliegenden Aufgaben werden durch die Gegenstände der unabhängigen Patentansprüche gelöst. Bevorzugte Ausführungsformen sind in den Unteransprüchen definiert.

Es wurde überraschend gefunden, daß der Zeaxanthin-Gehalt in transgenen Pflanzen durch die Inhibierung der Zeaxanthin-Epoxidase-Enzymaktivität in den transgenen Pflanzen erhöht werden kann. Dabei war insbesondere die Tatsache überraschend, dass der Zeaxanthingehalt in den transgenen Pflanzen weit über den ursprünglichen Gehalt an Violaxanthin hinaus erhöht ist.

Unter Inhibierung wird hierbei die verminderte Expression des Zeaxanthin-Epoxidase-Gens oder -Enzyms aufgrund der Expression eines sense- oder antisense-Konstrukts oder die aufgrund dessen verminderte Enzymaktivität oder eine aus anderen Gründen verminderte Enzymaktivität verstanden. Eine aus anderen Gründen verminderte Zeaxanthin-Epoxidase-Enzymaktivität wird bspw. durch die Expression von gegen Zeaxanthin-Epoxidase gerichteten Antikörpern oder die Expression bzw. Einführung von Zeaxanthin-Epoxidase-Inhibitoren erreicht. In jedem Fall bedeutet Inhibierung, dass die Zeaxanthin-Epoxidase-Gen/-Enzym-Expression im Vergleich zum Wildtyp vermindert ist, also nicht unbedingt ausgeschaltet ist; man kann auch von einer Down-Regulierung der endogenen Zeaxanthin-Epoxidase sprechen.

Dabei erfolgt die Inhibierung der Synthese der Zeaxanthin-Epoxidase vorzugsweise durch die Übertragung von rekombinanten Nukleinsäuremolekülen, die eine für Zeaxanthin-Epoxidase kodierende Nukleinsäuresequenz enthalten. In diesen rekombinanten Nukleinsäuremolekülen kann die für Zeaxanthin-Epoxidase kodierende Nukleinsäuresequenz entweder in Sense-Orientierung unter Kontrolle eines in Pflanzen aktiven Promotors vorliegen, was zur Co-Suppression der endogenen Zeaxanthin-Epoxidase-Enzymaktivität führt, oder die für eine Zeaxanthin-Epoxidase kodierende Nukleinsäuresequenz kann in Antisense-Orientierung unter Kontrolle eines in Pflanzen aktiven Promotors vorliegen. Die Übertragung von Antisense-Konstrukten führt zur Antisense-Inhibierung der endogenen Zeaxanthin-Epoxidase-Aktivität.

In beiden Fällen muß nicht zwingend die gesamte natürlicherweise für eine Zeaxanthin-Epoxidase kodierende Nukleinsäuresequenz übertragen und transkribiert werden, es können vielmehr auch Fragmente der kodierenden Region ausreichen, soweit diese Fragmente die Erzielung der gewünschten Effekte, d.h. Co-Suppression einerseits bzw. Antisense-Inhibierung andererseits gewährleisten. Der Fachmann kann z.B. geeignete Deletionskonstrukte herstellen und durch Übertragung auf transgene Pflanzenzellen prüfen, ob ein bestimmtes Fragment der kodierenden Region den erfindungsgemäßen Effekt, nämlich die Inhibierung der endogenen Zeaxanthin-Epoxidase-Aktivität hat oder nicht. Wie im Rahmen dieser Anmeldung verwendet, schließt die Formulierung "DNA-Sequenz, die für ein Protein mit der enzymatischen Aktivität einer Zeaxanthin-Epoxidase kodiert" somit auch solche DNA-Sequenzen und Fragmente ein, die zwar nicht für ein aktives Enzym kodieren, deren Anwesenheit und Transkription in Sense- oder Antisense-Orientierung aber eine Co-Suppression respektive Antisense-Inhibierung in transgenen Pflanzenzellen bewirkt. Man kann solche Fragmente im Zusammenhang mit der Erfindung auch als "Antisense- oder Suppressions-aktive" Zeaxanthin-Epoxidase-DNA-Fragmente bezeichnen.

Auch der vollständige genomische Klon einer Zeaxanthin-Epoxidase, also mit sämtlichen Introns, oder Teile davon können übertragen werden, um die gewünschte Inhibierung der endogenen Zeaxanthin-Epoxidase zu erreichen.

In jedem Fall ist der Begriff "antisense" dahingehend zu verstehen, daß die für eine Zeaxanthin-Epoxidase kodierende Sequenz bzw. ein "antisense-aktives" Fragment davon in antisense-Orientierung, also "verkehrt" herum in 3'->5'-Richtung, in operativer Verknüpfung mit einer in Pflanzenzellen aktiven Promotorregion vorliegt und transkribiert wird. Die RNA, die bei der Transkription eines derartigen Antisense-Gens entsteht, ist komplementär zur RNA des endogenen Gens und kann die Synthese des Zeaxanthin-Epoxidase-Proteinprodukts verhindern, indem es zur Hybridisierung zwischen der nativen und der Antisense-RNA kommt.

Im Falle des alternativ einsetzbaren Co-Suppressionskonstrukts liegt die für eine Zeaxanthin-Epoxidase kodierende Sequenz bzw. ein "Co-Suppressions-aktives" Fragment davon in sense-Orientierung (also in 5'->3'-Richtung) in operativer Verknüpfung mit einer in Pflanzenzellen aktiven Promotorregion vor. Bei dem Phänomen der Co-Suppression wird das endogene Gen durch zusätzlich eingeführte, identische (Teil)Kopien dieses Gens inaktiviert. Diese Inaktivierung ist vermutlich auf einen RNA-abhängigen Mechanismus zurückzuführen, an dem die RNA-dirigierte RNA-Polymerase beteiligt ist.

Weiter wurde überraschenderweise beobachtet, dass die Co-Suppression oder die Antisense-Inhibierung der endogenen Zeaxanthin-Epoxidase auch zu einer Erhöhung des Gesamt-Carotinoid-Gehalts führen kann. Dies bedeutet zum Beispiel im Falle transgener Kartoffelpflanzen, dass nicht nur der Gehalt an Zeaxanthin erhöht ist, sondern zusätzlich auch der Gehalt an Violaxanthin und Lutein. Allgemein bedeutet die Formulierung "Erhöhung des Gesamt-Carotinoid-Gehalts" im Rahmen dieser Anmeldung, daß neben dem Zeaxanthin-Gehalt der Gehalt von mindestens einem weiteren, in der Pflanzen natürlicherweise vorkommenden Carotinoid im Vergleich zur Wildtyppflanze erhöht ist, oder daß der Gehalt von mindestens einem, in der Pflanze natürlicherweise vorkommenden Carotinoid im Vergleich zur Wildtyppflanze erhöht ist, während der Zeaxanthin-Gehalt unverändert, oder erniedrigt ist.

Die Inhibierung oder Down-Regulierung der endogenen Zeaxanthin-Epoxidase-Aktivität kann auch auf andere Weise in transgenen Pflanzen oder anderen Wirtszellen/-organismen erreicht werden. Hier ist z.B. die Expression von gegen Zeaxanthin-Epoxidase gerichteten Antikörpern oder die Expression bzw. Einführung/Applikation von Zeaxanthin-Epoxidase-Inhibitoren zu nennen.

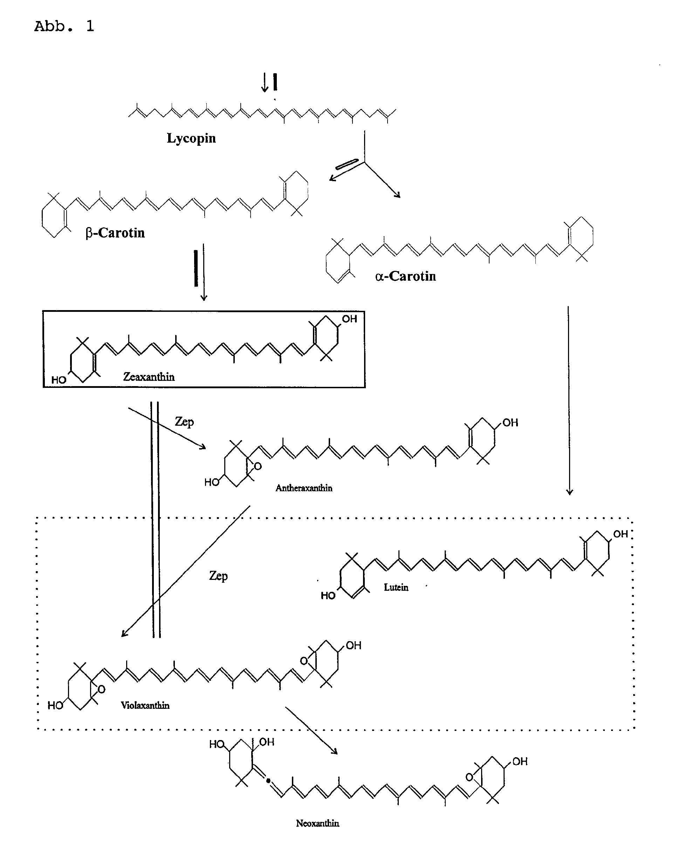

Das Enzym Zeaxanthin-Epoxidase katalysiert die Bildung des Carotinoids Violaxanthin aus Zeaxanthin (Sandmann (2001) vide supra). Abbildung 1 zeigt den Carotinoid-Biosyntheseweg in Kartoffelknollen, wobei die in Wildtyp-Knollen vertretenen Hauptcarotinoide Lutein und Violaxanthin (Iwanzik et al. (1983) Potato Res. 26, 149-162) durch einen gestrichelten Kasten hervorgehoben sind, während die Inaktivierung bzw. Inhibierung der Zeaxanthin-Epoxidase (Zep) durch eine doppelte Linie veranschaulicht wird. Die Inhibierung der Zeaxanthin-Epoxidase-Aktivität bzw. des Gehalts an aktiver Zeaxanthin-Epoxidase in transgenen Pflanzen führt zu einer verminderten Umsetzung von Zeaxanthin und somit zu einem erhöhten Zeaxanthin-Gehalt in den Pflanzen.

DNA-Sequenzen, die für ein Protein mit der enzymatischen Aktivität einer Zeaxanthin-Epoxidase kodieren, sind im Stand der Technik bekannt und stehen dem Fachmann zur Verfügung. Der Fachmann, der die der Erfindung zugrunde liegende überraschende Steigerung des Zeaxanthingehalts und ebenfalls die Steigerung des Gehalts an Gesamt-Carotinoiden erzielen möchte, ist somit mittels herkömmlicher Klonierungs-, Transformations- und Regenerationsmethoden in der Lage, transgene Pflanzen zu erzeugen, in denen der Zeaxanthin- und/oder Gesamt-Carotinoid-Gehalt z.B. durch Anwesenheit entsprechender Sense- oder Antisense-Konstrukte erhöht ist, zu erzeugen.

Neben der im Rahmen dieser Anmeldung erstmals bereitgestellten DNA-Sequenz aus Kartoffel (SEQ ID No. 1), stehen dem Fachmann weitere pflanzliche, für Zeaxanthin-Epoxidase kodierende DNA-Sequenzen zur Verfügung. Zum Beispiel aus Tomate (Acc.No. Z83835; Burbidge et al. (1997) J. Exp. Bot. 48:1749-1750), Tabak (*Nicotiana plumbaginifolia)* (Acc.No. X95732; Marin et al. (1996) EMBO J. 15:2331-2342), Paprika (Acc. No. X91491; Bouvier et al. (1996) J. Biol. Chem. 271:28861-28867), Aprikose (Acc.No. AF159948; Mbeguie-A-Mbeguie and Fils-Lycaon (2000) Plant Physiol. 122:291), *Arabidopsis thaliana* (Acc.No. AF281655) und Reis (Acc.No. AB050884; Agrawal et al. (2001) Plant Physiol. 125:1248-1257). Weitere für die Umsetzung der Erfindung geeignete Sequenzen kann der Fachmann gängigen Sequenz- oder Literaturdatenbanken entnehmen.

Bevorzugt erfolgt die Inhibierung der Zeaxanthin-Epoxidase-Aktivität gezielt in den Organen bzw. Geweben der Nutzpflanzen, die als Nahrung für Mensch und Tier Verwendung finden, wobei der Carotinoid-Stoffwechsel in den anderen Pflanzenorganen bzw. -geweben nach Möglichkeit nicht verändert ist, also dem Wildtyp entspricht. Von besonderem Interesse ist die Erhöhung des Zeaxanthin-Gehalts in Kartoffelpflanzen, wobei die Inhibierung der endogenen Zeaxanthin-Epoxidase-Aktivität bevorzugt nur in den Kartoffelknollen stattfindet, während die restlichen Teile der Pflanze im wesentlichen den Carotinoid-Phänotyp der Wildtyp-Pflanze zeigen. Im Falle der Kartoffel erfolgt die Transkription bzw. Expression der für Zeaxanthin-Epoxidase kodierenden Nukleinsäuresequenz daher in einer bevorzugten Ausführungsform unter Kontrolle eines Speicherorgan- bzw. Knollen-spezifischen Promotors.

Geeignete Promotoren sind dem Fachmann bekannt oder er kann sie üblichen Sequenz- und Literaturdatenbanken entnehmen. Als Promotoren für konstitutive Expression eignen sich beispielsweise der 35S RNA Promotor von Cauliflower Mosaic Virus (Frey et al. (1999) Plant Mol. Biol. 39:1267-1274) oder der Ubiquitin-Promotor (Drakakaki et al. (2000) Transgenic Res. 9:445-452).

Für die Knollen-spezifische Expression kann beispielsweise der Stärke-Synthase-Promotor aus Kartoffel verwendet werden (van der Steege et al. (1992) Plant Mol. Biol. 20, 19-30). Ein anderer für die Knollen-spezifische Expression geeigneter Promotoren ist beispielsweise der B33-Patatin-Promotor (Roche-Sosa et al. (1989) EMBO J. 8:23-29).

Beispiele für geeignete samenspezifische Promotoren sind der leb4-Promotor (Bäumlein et al. (1992) Plant J. 2:233-239), der napin-Promotor (Ellerström et al. (1996) Plant Mol. Biol. 32:1019-1027), der phaseolin-Promotor (Kawagoe et al. (1992) Plant J. 2:927-936), der in der deutschen Patentanmeldung DE 199 50 589 offenbarte Elongase-Promotor und der DC3-Promotor (Seffens et al. (1990) Dev. Genet. 11:65-76).

Samen-spezifische Promotoren wären besonders für die transgenen Pflanzen Raps, Mais und andere Getreide von Interesse.

Als Frucht-spezifische Promotoren eignen sich beispielsweise der cKPGA-Promotor (Wang et al. (2000) Plant Mol. Biol. 42:317-328), der RBCS1-3-Promotor (Meier et al. (1995) Plant Physiol. 107:1105-1118) und der 2A11-Promotor (Van Haaren et al. (1993) Plant Mol. Biol. 21:625-640).

Ein Frucht-spezifischer Promotor wäre besonders nützlich für Pflanzen wie Tomate und Paprika.

Des weiteren wären Wurzel-spezifische Promotoren (z.B. wie in WO 00/15662 beschrieben) für Pflanzen wie Rübe und Karotte vorteilhaft.

Auch andere Promotoren, wie bspw. induzierbare oder entwicklungsspezifische Promotoren können im Rahmen der Erfindung zum Einsatz kommen.

Ferner sind optional Transkriptions- und Terminationssequenzen vorhanden, die der korrekten Beendigung der Transkription dienen, sowie der Addition eines PolyA-Schwanzes an das Transkript dienen können, dem eine Funktion bei der Stabilisierung der Transkripte beigemessen wird. Derartige Elemente sind in der Literatur beschrieben und beliebig ausführbar. In der Regel sind solche Elemente bereits in den gängigen Expressionsvektoren in Form von Expressionskassetten enthalten.

Zur Vorbereitung der Einführung fremder Gene in höhere Pflanzen bzw. deren Zellen stehen eine große Anzahl von Klonierungsvektoren zur Verfügung, die ein Replikationssignal für *E. coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird dann für die Transformation von *E. coli*-Zellen verwendet. Transformierte *E. coli*-Zellen werden in einem geeigneten Medium gezüchtet und anschließend geerntet und lysiert, und das Plasmid wird wiedergewonnen. Als Analysenmethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und gewonnene DNA-Fragmente mit anderen DNA-Sequenzen verknüpft werden. Bei sämtlichen Klonierungsschritten sowie bei der Herstellung und Analyse der transgenen Mikroorganismen bzw. Pflanzen kann der Fachmann auf geläufige Klonierungs-, Hybridisierungs, Sequenzierungsund Transformationsmethoden sowie PCR-Techniken zurückgreifen, die in jedem bio- oder gentechnologischen Labor wohl bekannt und etabliert sind (s. bspw. Sambrook et al. (1989) Molecular Cloning: A Laboratory Manual, 2. Auflage, Cold Spring Harbour Laboratory Press, Cold Spring Harbour, New York).

Voraussetzung für die Einführung der erfindungsgemäßen rekombinanten Nukleinsäuremoleküle und Vektoren in Pflanzenzellen ist die Verfügbarkeit geeigneter Transformationssysteme. Hier wurde während der letzten zwei Jahrzehnte ein breites Spektrum an Transformationsmethoden entwickelt und etabliert. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, den direkten Gentransfer isolierter DNA in Protoplasten, die Injektion und Elektroporation von DNA in Pflanzenzellen, die Einbringung von DNA mittels biolistischer Methoden sowie weitere Möglichkeiten, wobei der Fachmann die jeweils geeignete Methode problemlos ermitteln kann. Sämtliche Transformationsverfahren sind seit vielen Jahren gut etabliert und gehören zweifelsohne zum Standard-Repertoire des Fachmanns in der Pflanzenmolekularbiologie, Pflanzenbiotechnologie und Zell- und Gewebekultur.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide wie z.B. pUC-Derivate, verwendet werden. Sollen aber aus derartig transformierten Zellen ganze Pflanzen regeneriert werden, ist die Anwesenheit eines selektierbaren Markergens empfehlenswert. Dem Fachmann sind die gängigen Selektionsmarker bekannt, und es stellt für ihn kein Problem dar, einen geeigneten Marker auszuwählen.

Je nach Transformationsmethode können zusätziche DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der im Ti- bzw. Ri-Plasmid enthaltenen T-DNA als Flankenbereich mit den einzuführenden Genen verbunden werden. Werden für die Transformation Agrobakterien verwendet, muß die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E. coli* als auch in Agrobakterien replizieren. Sie enthalten ein Selektions-Markergen und einen Linker oder Polylinker, welche von der rechten und linken T-DNA-Grenzregion eingerahmt werden. Sie können direkt in die Agrobakterien transformiert werden. Das als Wirtszelle dienende Agrobakterium soll ein Plasmid enthalten, das eine vir-Region trägt. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet. Die Verwendung von T-DNA für die Transformation von Pflanzenzellen ist intensiv untersucht und ausreichend in allseits bekannten Übersichtsartikeln und Handbüchern zur Pflanzentransformation beschrieben worden.

Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin, Methotrexat, Glyphosat, Streptomycin, Sulfonylharnstoff, Gentamycin oder Phosphinotricin u.a. vermittelt. Der individuell gewählte Marker sollte daher die Selektion transformierter Zellen gegenüber Zellen, denen die eingeführte DNA fehlt, gestatten. Hierzu sind auch alternative Marker geeignet, wie nutritive Marker, Screening-Marker (wie GFP, green fluorescent protein). Selbstverständlich kann auch vollkommen auf Selektionsmarker verzichtet werden, was allerdings mit einem ziemlich hohen Screening-Bedarf einhergeht. Falls der angesetzte Selektions-Marker nach erfolgter Transformation und Identifizierung erfolgreich transformierter Zeller bzw. Pflanzen wieder entfernt werden soll, also Marker-freie Pflanzen erwünscht sind, stehen dem Fachmann auch hierfür verschiedene Strategien zur Verfügung. So können z.B. sequenzspezifische Rekombinasen verwendet werden, z.B. in Form der Retransformation einer Rekombinaseexprimierenden Ausgangslinie und Auskreuzung der Rekombinase nach erfolgter Entfernung des Selektionsmarkers (siehe z.B. Reiss et al. (1996) Proc. Natl. Acad. Sci. USA 93, 3094-3098; Bayley et al. (1992) Plant Mol. Biol. 18, 353-361; Lloyd et al. (1994) Mol. Gen. Genet. 242, 653-657; Mäser et al. (1991) Mol. Gen. Genet. 230, 170-176; Onouchi et al. (1991) Nucl. Acids Res. 19, 6373-6378). Der Selektionsmarker kann auch durch Cotransformation mit anschließender Auskreuzung entfernt werden.

Die Regeneration der transgenen Pflanzen aus transgenen Pflanzenzellen erfolgt nach üblichen Regenerationsmethoden unter Verwendung bekannter Nährmedien und ggf. üblicher Phytohormone. Die so erhaltenen Pflanzen können dann, falls erwünscht, mittels üblicher Verfahren, einschließlich molekularbiologischer Methoden, wie PCR, Blot-Analysen oder biochemischer Verfahren auf Anwesenheit der eingeführten DNA, die ein Protein mit enzymatischer Aktivität einer Zeaxanthin-Epoxidase kodiert, untersucht werden. Alternativ kann natürlich auch der Carotinoid-Phänotyp der transgenen Pflanze anhand der endogenen Zeaxanthin-Epoxidase-Aktivität untersucht werden.

Es ist klar, daß in jedem Transformationsexperiment Transformanden mit unterschiedlicher Kopienzahl des eingeführten Genkonstrukts und in der Regel auch mit unterschiedlichen Expressionsraten bezüglich des eingeführten Konstrukts entstehen und selektiert werden. Für z.T. recht große Unterschiede in den Expressionsraten sind vor allem Positionseffekt verantwortlich, also die Lage der eingeführten DNA innerhalb des pflanzlichen Genoms. Der mit solchen Phänomenen vertraute Fachmann kann die für ihn je nach Zielsetzung geeigneten transgenen Pflanzen aus einem oder mehreren Transformationsexperimenten mittels einfacher Techniken (z.B. Blot-Analysen, Bestimmung des Carotinoid-Gehalts, etc.) auswählen. Wenn also eine Transformande mit einem besonders hohen Zeaxanthin-Gehalt erwünscht ist, wird der Fachmann sich auf solche Transformanden konzentrieren und diese für die Erzeugung von Nachkommen, für Rückkreuzungen, für die Erzeugung homozygoter Tochtergenerationen u.a. Zwecke einsetzen.

Bei der transgenen Pflanze bzw. den transgenen Pflanzenzellen, in denen der Zeaxanthinund/oder Gesamt-Carotinoid-Gehalt durch Inhibierung der endogenen Zeaxanthin-Epoxidation erhöht ist, kann es sich um jede beliebige monokotyle oder dikotyle Pflanze bzw. Pflanzenzellen handeln, vorzugsweise handelt es sich um Nutzpflanzen bzw. Zellen von Nutzpflanzen mit beliebigem Zeaxanthingehalt oder Nutzpflanzen bzw. Zellen von Nutzpflanzen, die natürlicherweise einen unerwünscht niedrigen Zeaxanthin-Gehalt aufweisen. Ebenfalls bevorzugt sind Getreide, wie Weizen, Gerste, Roggen, Reis und Mais, Raps, Zuckerrübe oder Soja. Besonders bevorzugt handelt es sich um Pflanzen mit Speicherorganen wie z.B. Kartoffel. Prinzipiell ist aber jede Nutzpflanze für die Umsetzung der Erfindung erstrebenswert, die für Ernährungszwecke geeignet ist und somit dazu beitragen kann, den Zeaxanthin-Gehalt in der Nahrung für Mensch und Tier zu verbessern.

Die Erfindung betrifft ebenfalls Ernteprodukte und Vermehrungsmaterial der erfindungsgemäßen Pflanzen, beispielsweise Früchte, Samen, Knollen, Wurzelstücke, Sämlinge, Stecklinge etc.

In einer bevorzugten Ausführungsform exprimieren die transgenen Pflanzen bzw. Pflanzenzellen, insbesondere transgenen Kartoffelpflanzen bzw. -zellen, neben den oben erwähnten Zeaxanthin-Epoxidase-Konstrukten mindestens ein weiteres eingeführtes Gen. Bei diesem Gen bzw. Nukleinsäure handelt es sich einerseits um Gene bzw. Nukleinsäuren, die für ein Enzym kodieren, das einen der Zeaxanthin-Epoxidase vorgelagerten Schritt des Carotinoid-Stoffwechsels katalysiert, andererseits um Gene bzw. Nukleinsäuren, die für ein Enzym kodieren, das einen der Zeaxanthin-Epoxidase nachgelagerten Stoffwechselschritt katalysieren. Im ersten Fall wird durch das Erhöhen der Konzentration an Zeaxanthin- bzw. Carotinoid-Vorläufern der Gehalt an Zeaxanthin bzw. Gesamt-Carotinoid zusätzlich erhöht. Beispiele für Gene, die sich hierfür eignen, kodieren für Phytoen-Synthase, Phytoen-Desaturase, ζ-Carotin-Desaturase, Lycopin-β-Cyclase, β-Carotin-Hydroxylase, Lycopin-α-Cyclase, β-Carotin-Diketolase (z.B. auch bakteriell oder aus Algen), α-Carotin-Hydroxylase. Entsprechende Sequenzen kann der Fachmann den gängigen Zeitschriften bzw. Sequenzdatenbanken entnehmen.

Durch Co-Transformation der erfindungsgemäßen Pflanzen bzw. Pflanzenzellen mit Genen bzw. Nukleinsäuren, die für Enzyme des der Zeaxanthin-Epoxidase nachgelagerten Stoffwechsels kodieren, wird erreicht, daß das angereicherte Zeaxanthin bzw. die angereicherten Gesamt-Carotinoide in verstärktem Maß in im Stoffwechsel nachgeschaltete Produkte umgewandelt werden. Hier sind als Beispiele zu nennen Nukleinsäuren, die für Violaxanthin-Dioxygenase, Zeaxanthin-Ketolase, Neoxanthin-Synthase und Capsanthin-Synthase kodieren. Auch hier sind geeignete Sequenzen dem Fachmann zugänglich.

Weiter eignen sich für die Co-Transformation und -Expression Gene, die für Enzyme kodieren, die frühe Schritte der Synthese und Umwandlung von Prenyl-Pyrophosphaten katalysieren. Hier sind beispielhaft zu nennen: 1-Deoxyxylulose-5-phosphat-Synthase, 1-Deoxyxylulose-5-phosphat-Reductoisomerase, Isopentenylpyrophosphat-Isomerase und Geranylgeranylpyrophosphat-Synthase. Diese Enzyme limitieren die Carotinoidbiosynthese in *E. coli.*

Der Fachmann ist mit der Co-Expression von zwei oder mehr Genen bzw. Genkonstrukten in transgenen Organismen wie Pflanzen vertraut. So können bspw. verschiedene DNA-Konstrukte mit unterschiedlichen Selektionsmarkern auf Pflanzenzellen gleichzeitig oder nacheinander übertragen werden. Im Falle von Pflanzen eignet sich natürlich auch die Kreuzung, um Pflanzen mit dem gewünschten Genotyp herzustellen.

Obgleich in obiger Beschreibung in der Regel auf die Erhöhung des Zeaxanthin-Gehalts bzw. des Gehalts an Gesamt-Carotinoiden in Pflanzen/-zellen Bezug genommen wird, sind die erfindungsgemäßen Verfahren zur Erhöhung des Zeaxanthin-Gehalts bzw. des Gehalts an Gesamt-Carotinoiden auch auf andere Wirtszellen/-organismen wie bspw. Algen und vor allem auch auf pflanzliche Zellkulturen anwendbar. Solche Zellen/Organismen bzw. Kulturen eignen sich hervorragend als Produktionsstätte für Zeaxanthin und die Gewinnung von Zeaxanthin als Nahrungsergänzung.

Die Erfindung wird in den nachfolgenden Beispielen erläutert.

### Beispiele

### Herstellung von Sense- und Antisense-Konstrukten

Ein cDNA-Fragment, dass für Zeaxanthin-Epoxidase aus Kartoffel kodiert, wurde in den mit pBin19 (Bevan (1984) Nucl. Acids Res. 12, 8711-8721) verwandten Vektor pPGB121S (van der Steege et al. (1992) Plant Mol. Biol. 20, 19-30) in Sense- und in Antisense-Orientierung kloniert.

Hierzu wurde zunächst Gesamt-RNA aus Blättern von sterilen Kartoffelpflanzen (*Solanum tuberosum* L. cv. Desiree) nach Kuntz et al. isoliert (1992 Plant J. 21, 25-34). 2 µg RNA wurden mit M-MuLV Reverse-Transkriptase (MBI Fermentas, St. Leon-Rot, Deutschland) in Gegenwart von oligo-dT-Primern reverse-transkribiert. Die erhaltene cDNA wurde gereinigt (PCR Purification-Kit von Roche, Mannheim, Deutschland) und als Template für PCR-Amplifizierung eines cDNA-Fragments, welches für Zeaxanthin-Epoxidase kodiert, eingesetzt. Die PCR wurde mit folgenden degenerierten Oligonukleotiden als Primer in einem Thermocycler (PCT-100, MJ Research Inc., Watertown, USA) durchgeführt: Primer zep1: 5′-TGG TAY TGY AAR TTY GAY ACI TTY AC-3′ und Primer zep2: 5′-GCR TCR TCR TCY TCR  AAC CAI-3′. Y bedeutet, daß die Positon durch C oder T besetzt sein kann; R steht für A oder G und I für Inosin.

Das PCR-Protokoll war wie folgt: 3 min. Denaturierung gefolgt von 30 Zyklen folgender Abfolge: 1 min 95°C, 1 min 52°C, 1 min 72°C, mit einer abschließenden Synthese bei 72°C für 5 min und anschließendem Kühlungsschritt bei 4°C. Das PCR-Produkt (1000 bp) wurde direkt in den Vektor pCR2.1 (Invitrogen, Carlsbad, CA, USA) nach Herstellerangaben kloniert und das subklonierte Fragment durch Sequenzierung nach Sanger verifiziert.

Zwei Zeaxanthin-Epoxidase-cDNA-Fragmente in entgegengesetzter Orientierung wurden aus dem Plasmid mittels Restriktionsverdau mit BamHI und XbaI isoliert und nach Agarosegelelektrophorese aus dem Gel gereinigt. Die Fragmente wurden anschließend in den mit BamHI und XbaI verdauten binären Vektor pPGB 121S kloniert. Dieser Vektor enthält den Granulebound Stärkesynthase-Promotor aus Kartoffel (van der Steege et al. (1992) vide supra) und führt somit zu einer Knollen-spezifischen Expression des eingefügten cDNA-Fragments bei Übertragung auf Kartoffelpflanzen. 3' vom eingefügten cDNA-Fragment liegt die Nopalin-Synthase-Terminatorsequenz.

Die erhaltenen Konstrukte pPGB-zep-sense - hier steht der Zeaxanthin-Epoxidase-cDNA-Klon in Sense-Orientierung unter Kontrolle des knollenspezifischen Promotors aus Kartoffel - und pPGB-zep-anti - hier steht der Zeaxanthin-Epoxidase-cDNA-Klon in Antisense-Orientierung unter Kontrolle des knollenspezifischen Promotors aus Kartoffel - wurden durch Restriktionsverdaue und Sequenzierung verifiziert.

Der aus Kartoffel isolierte, für eine Zeaxanthin-Epoxidase kodierende cDNA-Klon ist als SEQ ID No. 1 beigefügt. Die cDNA umfasst 1044 Basenpaare und einen ORF von 348 Aminosäuren. Die abgeleitete Aminosäuresequenz ist der beigefügten SEQ ID No. 2 zu entnehmen.

### Herstellung transgener Kartoffelpflanzen

Für die Pflanzentransformation wurden *Solanum tuberosum* L. cv. Baltica und cv. Freya (SaKa-Ragis Pflanzenzucht GbR, Windeby, Deutschland) eingesetzt, wobei auch andere Kartoffellinien verwendet werden können. Pflanzen wurden in Gewebekultur mit einem 16 h Licht/8 h Dunkelheit-Rhythmus auf MS-Medium (Murashige and Skoog (1962) Physiol. Plant. 15, 473-497) mit 2% Saccharose gehalten. Im Gewächshaus wurden die Pflanzen unter den gleichen Bedingungen mit zusätzlichem künstlichen Licht unter einem Minimum von 250 µmol Photonen m² sec⁻¹ kultiviert.

Die Kontrukte pPGB-zep-sense und pPGB-zep-anti wurden nach dem Protokoll von Römer et al. (2000, vide supra) in *Agrobacterium tumefaciens* LBA 4404 eingeführt. Anschließend wurden die Kontrukte über Agrobakterium-vermittelten Gentransfer auf Kartoffelpflanzen (Baltica bzw. Freya) übertragen (Rocha-Sosa et al. (1989) EMBO J. 8, 23-29). Transgene Pflanzen wurden auf Kanamycin-haltigem Medium selektioniert (Dietze et al. (1995) In: Gene Transfer to Plants XXII (Potrykus and Spangenberg, eds). Berlin: Springer Verlag pp 24-29). Stabile Transformation von unabhängigen regenerierten Pflanzen wurde mittels PCR verifiziert, und nach Vermehrung wurden jeweils drei Pflanzen pro Linie in das Gewächshaus überführt, um Miniknollen zu erhalten. Aus sechs Transformationsreihen wurden insgesamt 127 unabhängige Transformanden erhalten, die in das Gewächshaus überführt und in 2 l-Töpfen kultiviert wurden.

Reife Miniknollen wurden geerntet, zunächst hinsichtlich ihrer Farbe im frischen Zustand untersucht und anschließend hinsichtlich der Carotinoid-Zusammensetzung der Knolle analysiert. Da die erwünschten Knollen solche mit erhöhtem Zeaxanthin-Gehalt waren, wurde nur der Carotinoind-Gehalt der positiven Linien in untenstehender Tabelle 1 aufgelistet.

**Tabelle 1**

| Carotinoid-Gehalt von transgenen Kartoffellinien (µg/g Trockengewicht) | | | | | | | | |
|---|---|---|---|---|---|---|---|---|
| Pflanze* | Gesamtcarotinoide | Neox | Viol | Anth | Lut | Zeax | βCry | βCar |
| 47-WT | 10.6 | 0.6 | 3.5 | 0.3 | 2.7 | 0.3 | 0.6 | 0.7 |
| 47-17 | 35.0 | 0 | 0.1 | 1.5 | 1.4 | 29.5 | 1.2 | 1.3 |
| 47-18 | 60.8 | 0.7 | 1.7 | 14.9 | 5.2 | 40.1 | 2.3 | 2.4 |
| 47-36 | 35.5 | 1.7 | 16.2 | 4.5 | 7.3 | 1.4 | 1.4 | 2.6 |
| 47-51 | 31.8 | 0.7 | 5.1 | 8.6 | 3.2 | 11.3 | 0.7 | 1.9 |
| 47-52 | 33.9 | 1.8 | 15.4 | 3.0 | 7.0 | 1.4 | 1.3 | 1.2 |
| 47-53 | 44.3 | 0.7 | 4.0 | 12.8 | 2.0 | 22.5 | 1.0 | 1.3 |
| 47-73 | 50.7 | 1.7 | 7.6 | 16.3 | 5.0 | 18.7 | 0.5 | 1.0 |
| | | | | | | | | |
| 48-WT | 20.4 | 2.0 | 12.2 | 0.5 | 5.4 | 0.3 | 0.6 | 0.4 |
| 48-5 | 27.2 | 1.0 | 13.2 | 1.1 | 7.3 | 1.5 | 1.7 | 1.6 |
| 48-17 | 43.2 | 1.3 | 10.7 | 12.1 | 2.6 | 16.5 | 0.5 | 0.4 |
| 48-18 | 26.6 | 1.2 | 14.1 | 1.9 | 5.8 | 2.5 | 0.6 | 0.6 |
| 48-19 | 25.1 | 0.8 | 11.5 | 1.9 | 6.6 | 2.7 | 0.8 | 0.9 |
| 48-20 | 42.2 | 0.5 | 4.4 | 13.2 | 3.4 | 1.7 | 1.6 | 1.8 |
| 48-27 | 19.0 | 0.5 | 5.3 | 0.9 | 6.0 | 0.7 | 2.6 | 3.0 |
| 48-36 | 43.0 | 1.4 | 22.4 | 2.0 | 11.6 | 2.4 | 1.5 | 1.7 |
| 48-39 | 38.3 | 1.2 | 18.1 | 2.4 | 10.2 | 4.3 | 0.5 | 1.5 |
| | | | | | | | | |
| 49-WT | 20.0 | 1.0 | 7.4 | 0.7 | 5.3 | 0.2 | 1.6 | 1.6 |
| 49-2 | 24.9 | 0.8 | 9.7 | 2.2 | 5.5 | 0.8 | 1.5 | 1.9 |
| 49-4 | 35.4 | 1.9 | 16.6 | 4.5 | 5.8 | 1.5 | 1.1 | 2.2 |
| | | | | | | | | |
| 50-WT | 23.0 | 1.2 | 9.4 | 1.1 | 5.5 | 1.3 | 1.6 | 2.9 |
| 50-12 | 41.7 | 1.1 | 11.9 | 4.4 | 13.4 | 7.0 | 3.1 | 0.8 |
| 50-24 | 32.2 | 1.5 | 13.6 | 1.0 | 6.8 | 6.6 | 1.4 | 1.4 |
| | | | | | | | | |
| 52-WT | 22.2 | 0.7 | 11.1 | 0.7 | 6.9 | 0.8 | 1.2 | 0.9 |
| 52-7 | 33.8 | 0.8 | 14.1 | 1.8 | 8.5 | 3.0 | 2.7 | 2.9 |
| 52-13 | 42.2 | 1.3 | 19.4 | 7.5 | 7.2 | 3.9 | 0.7 | 2.1 |
| 52-25 | 53.2 | 2.0 | 27.2 | 5.8 | 10.0 | 2.9 | 1.4 | 3.4 |
| 52-26 | 41.1 | 0.0 | 13.5 | 12.9 | 5.1 | 7.0 | 0.3 | 1.4 |

| | | | | | | | | |
|---|---|---|---|---|---|---|---|---|
| * Reihe 47 (Baltica, Co-Suppression) mit 26 getesteten transgenen Linien, Reihe 48 (Baltica, Antisense-Inhibierung) mit 37 getesteten transgenen Linien, Reihe 49 (Freya, Co-Suppression) mit 10 getesteten transgenen Linien, Reihe 50 (Freya, Antisense-Inhibierung) mit 6 getesteten transgenen Linien, Reihe 52 (Baltica, Antisense-Inhibierung) mit 22 getesteten transgenen Linien, und Reihe 53 (Baltica, Co-Suppression) mit 26 getesteten transgenen Linien. WT steht für nicht-trangene Kontrollinien. Neox = Neoxanthin Viol = Violaxanthin Anth = Antheraxanthin Lut = Luthein Zeax = Zeaxanthin βCry = β-Cryptoxanthin βCar = β-Carotin | | | | | | | | |

Im Vergleich zu Wildtyp-Pflanzen variierte der Zeaxanthin-Gehalt innerhalb der Transformationsreihe 47 (Baltica, Co-Suppression, Sense-Konstrukt) von 4- bis 134-fach. Ebenso in der Reihe 48 (Baltica, Antisense-Konstrukt) war der höchste Zeaxanthin-Wert 55-fach höher als in der entsprechenden Kontrollpflanzen. Insgesamt ergab die Reihe 47 26 Zeaxanthin-Transformanden, die Reihe 48 erzielte 37 Zeaxanthin-Transformanden und die Transformationsreihe 49 (Freya, Co-Suppression, Sense-Konstrukt) resultierte in 10 Zeaxanthin-Transformanden. In der Reihe 50 (Freya, Antisense) und der Reihe 52 (Baltica, Antisense) zeigten nur einige positive Linien einen mäßig erhöhten Zeaxanthin-Gehalt, nämlich eine Steigerung von 4- bis 7,5-fach gegenüber dem Wildtyp. In der Reihe 53 (Baltica, sense-Orientierung, Co-Suppression) wurde keine Linie mit gegenüber Wildtyp erhöhtem Zeaxanthin-Gehalt erhalten. Dies ist u.a. darauf zurückzuführen, dass für die letztgenannten Transformationsreihen nur eine relativ kleine Anzahl von Primärtransformanden mit integriertem Genkonstrukt erhalten wurden und somit analysiert werden konnten.

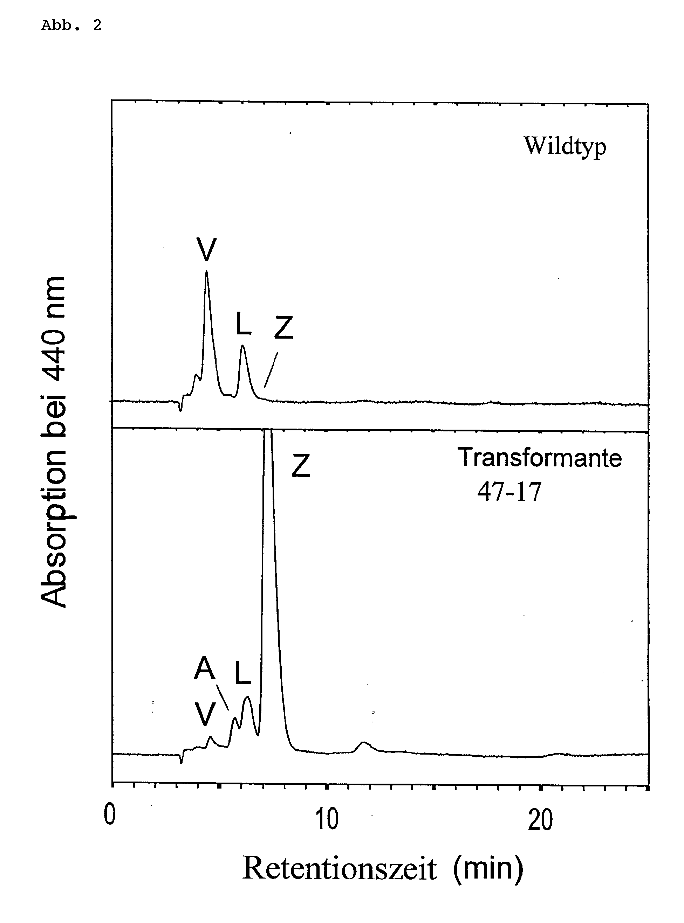

In Abbildung 2 ist eine HPLC-Trennung auf der Vydac 218TP54-Säule der Wildtypkontrolle aus der Reihe 47 (oben) und der transgenen Linie 47-17 (unten) gezeigt. In der Wildtyp-Knolle ist Violaxanthin das dominierende Carotinoid und auch große Mengen an Lutein wurden gemessen. Des weiteren konnten Spuren von Neoxanthin, Antheraxanthin, Zeaxanthin, β-Cryptoxanthin und β-Carotin in den Knollen der Kontrolle gefunden werden. In der positiven Transformande 47-17, die hier als Beispiel gezeigt wird, entspricht der Lutein-Gehalt (L) zwar in etwa der der Kontrolle, aber der Violaxanthin-Peak (V) ist deutlich verringert, während der Peak von Antheraxanthin (A) erhöht ist. Eindeutig ist in jedem Fall, dass der Zeaxanthin-Gehalt in der transgenen Pflanze im Vergleich zum Wildtyp drastisch erhöht ist, Zeaxanthin (Z) ist zweifelsohne das dominierende Carotinoid in dieser Transformande.

### Carotinoid-Analyse

Zwei bis vier einzelne Hälften von Miniknollen (1,5 bis 4 cm Länge) wurden gefriergetrocknet. Das gesamte Material wurde ohne die Schale durch ein Sieb (425 µm Porengröße) gerieben. Zu 150 mg des feinen Pulvers wurden 25 ml Aceton hinzugegeben und für 20 min auf 50°C erwärmt. Es wurde mit 10% Diethylether in Petrol extrahiert und der Gesamtcarotinoid-Gehalt bei 450 nm aus der oberen Phase bestimmt. Die gesamte obere Phase wurde in Stickstoff verdampft, der Rückstand erneut in Aceton gelöst und die Carotinoide mittels HPLC aufgetrennt. Die HPLC wurde auf einer 25 cm C18 Vydac 218TP54-Säule mit Methanol als mobile Phase (Eppler et al. (1992) J. Chromatogr. 20, 89-101) oder auf einer 25 cm Spherisorb ODS-1 mit einem Acetonitril/Methanol-Gradienten, wie von Gilmore and Yamamoto (1991, J. Chromatogr. 543, 137-145) beschrieben, durchgerührt.

Die Verteilung der einzelnen Carotinoide wurde durch Integration der Peak-Bereiche und der zur Berechnung der Gehalte an verschiedenen Carotinoiden eingesetzten Gesamtmengen erhalten. Referenz-Carotinoide wurden aus Cyanobakterien (Steiger et al. (1999) J. Photochem. Photobiol. B521, 14-18) oder Paprikablättern (Simkin et al. (2000) J. Agric. Food Chem. 48, 4676-4680) erhalten.

Die Bestimmung des Gesamtcarotinoid-Gehalts ergab, dass die Menge an Carotinoiden in verschiedenen Transformanden bis zu 5,7-fach höher ist als in den Wildtyp-Kontrollpflanzen. Auch dieser Effekt war ohne Zweifel äußert überraschend. Bei einer Gruppe von Transformanden war der Carotinoid-Gehalt erhöht, obwohl der Zeaxanthin-Gehalt nicht erhöht war, während bei einer zweiten Gruppe sowohl der Gehalt an Gesamt-Carotinoiden als auch der Gehalt an Zeaxanthin erhöht waren.

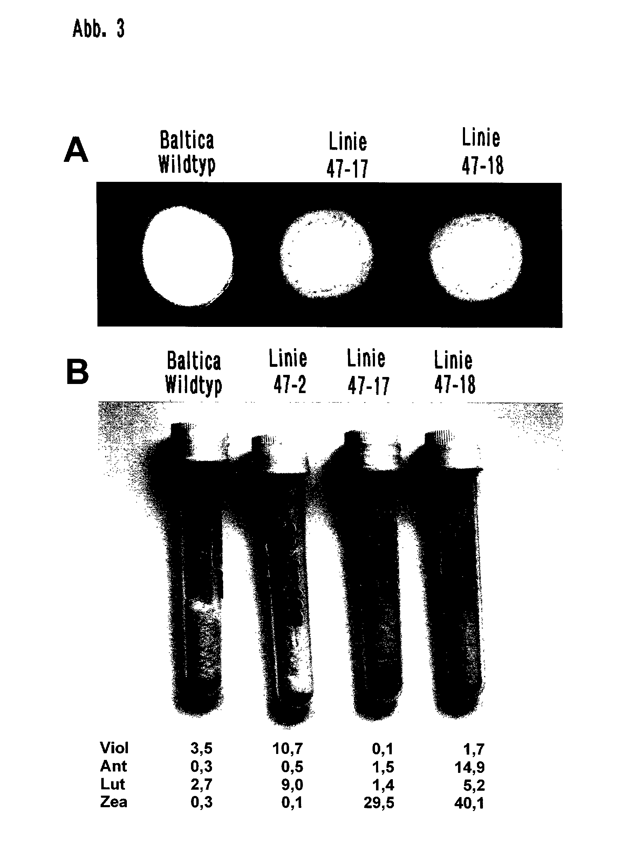

Der Effekt des erhöhten Zeaxanthin-Gehalts war sowohl an den Kartoffelknollen selbst sowie anhand des pulverisierten Materials eindeutig erkennbar (Abbildung 3). Die transgenen Kartoffelknollen (z.B. 47-17 und 47-18) zeigten bereits bei Betrachtung der Knollenhälften im Vergleich zum Wildtyp (z.B. Baltica) eine dunklere gelbe bis orangefarbene Farbe. Die Untersuchung von pulverisiertem Material der Linien 47-2, 47-17 und 47-18 im Vergleich zu Wildtyp-Material (Baltica) ergab zudem, dass eine intensivere Farbe des Knollenfleisches sowohl in Linien mit erhöhten Zeaxanthin-Konzentrationen (47-17, 47-18) als auch in solchen Linien ohne erhöhte Zeaxanthin-Akkumulation (47-2) beobachtet werden konnte. In der letztgenannten Linie waren Violaxanthin und Lutein die angereicherten Carotinoide. Der höchste Zeaxanthin-Gehalt wurde in der Transformande 47-18 mit einem Wert von 40,1 µg/g Trockengewicht gefunden (siehe Tabelle 1).

Aus Tabelle 1 ist ersichtlich, dass die gefundenen und analysierten Transformanden verschiedenen Typen zugeordnet werden können, die sich hinsichtlich ihrer quantitativen Carotinoid-Zusammensetzung unterscheiden. Diese Beobachtung deutet auf eine differentielle Beeinflussung der Regulation des Carotinoid-Stoffwechsels in den Transformanden hin. Danach kann man die Transformanden anhand ihrer quantitativen Carotinoid-Zusammensetzung in drei Gruppen einteilen. Linien der Gruppe I zeigen eine verstärkte Bildung aller Carotinoide, also nicht nur von Zeaxanthin, sondern auch von Violaxanthin und Lutein. Beispiele für nach und nach erhöhte Carotinoidsynthese sind die Linien 47-52, 50-12 und 48-36. Offensichtlich ist die Epoxidase-Reaktion in diesen Linien nur leicht inaktiviert und der Haupteffekt besteht darin, dass die Precursor in zyklische Carotinoide einfließen, die mehr oder weniger die Pools aller nachfolgenden Carotinoide erhöhen (Abbildung 1, angedeutet durch schwarze Balken).

In anderen Linien ist der Zeaxanthin-Gehalt stark erhöht, während das Epoxidationsprodukt des Zeaxanthins, Violaxanthin, erniedrigt ist und Lutein ungefähr konstant bleibt. Der Antheraxanthin-Gehalt bleibt entweder niedrig (Gruppe II, z.B. Linie 47-17) oder ist stark erhöht (Gruppe III, z.B. Linien 47-18, 47-45). In diesen Fällen wird die relativ starke Inaktivierung der Epoxidation durch eine Up-Regulierung des gesamten Carotinoid-Stoffwechsels überlagert. Dieser verstärkte Einstrom in den Stoffwechselweg wird bevorzugt in den β-Carotin-Zweig des Stoffwechsels geleitet (Abbildung 1, angezeigt durch einen offenen Balken), was darauf hin deutet, dass Violaxanthin in der Regulation der Carotinoid-Biosynthese in Kartoffelknollen eine wichtige Rolle spielt. Während einer starken, aber nicht vollständigen Inaktivierung der Zeaxanthin-Epoxidase-Expression (Zep) (Abbildung 1, doppelte Linie) verbleiben nur geringe Mengen an Enzym , die nur den ersten Epoxidationsschritt zu Antheraxanthin mit nur einer Epoxy-Gruppe katalysieren können (Linien 47-18, 47-45). Wenn die Epoxidase-Expression noch stärker unterdrückt wird, wird auch die Bildung von Antheraxanthin negativ beeinflusst (Linie 47-17). Innerhalb der Gruppe II wurde die Linie 47-18 als die Linie mit dem höchsten Zeaxanthin-Gehalt gefunden.

Dank der Erfindung und der hierdurch jetzt erstmals möglichen Erhöhung des Zeaxanthin-Gehalts in transgenen Pflanzen, insbesondere in transgenen Kartoffelknollen, kann der Nährwert pflanzlicher Nahrungsmitteln erheblich verbessert werden. Eine einzelne Kartoffelknolle durchschnittlicher Größe kann unsere Ernährung um ungefähr 0,6 mg Zeaxanthin bereichern, wenn z.B. eine Knolle der in Tabelle 1 angegebenen transgenen Baltica-Linien verwendet wird. Nach den Ergebnissen der Eye Disease Case Control Study Group (Seddon et al. (1994) J. Am. Diet. Assoc. 273, 1413-1420) bietet der Verzehr der transgenen Kartoffelknollen unmittelbar als Nahrungsmittel oder nach der Verarbeitung der Knollen zu verschiedenen Kartoffelprodukten zusammen mit anderen Lutein-reichen Gemüsen eine gute Möglichkeit, das Risiko altersbedingter Makulardegeneration zu vermeiden.

### Abbildungen

### Abbildung 1

Carotinoid-Biosyntheseweg in Kartoffelknollen. Die Inhibierung der Zeaxanthin-Epoxidase (Zep) ist durch eine doppelte Linie angedeutet.

### Abbildung 2

HPLC-Profil von Kartoffelknollen vom Wildtyp und von Linie 47-17. Die Trennung wurde auf einer C18 Vydac 218TP54-Säule mit Methanol als mobile Phase durchgeführt.

### Abbildung 3

A) Fleischfarbe von Miniknollen, erhalten von Baltica-Wildtyppflanzen, von der transgenen Linie 47-17 und von der transgenen Linie 47-18.
B) Färbung von gefriergetrocknetem Kartoffelpulver vom Wildtyp und von den transgenen Linien 47-2, 47-17 und 47-18. Der Gehalt der wichtigsten Carotinoide ist darunter in µg/g Trockengewicht angegeben. Viol: Violaxanthin, Ant: Antheraxanthin, Lut: Lutein, Zea: Zeaxanthin.

Sequenzprotokoll

SEQ ID No. 1

TGGTACTGTA AGTTTGACAC GTTCACTCCA GCAGTGGAAC GTGGACTTCC
CGTGGCAAGA GTCATCAGCC GCATGACTTT GCAGCAGATC CTTGCACGTG
CTGTTGGGGA GGATACAATT ATGAATGAAA GTAATGTTGT AGACTTTGAG
GATGATGGGG AGAAGGTTAG TGTGGTTATT GAGAATGGAC AACGATTTAC
AGGTGATCTT CTGGTTGGTG CTGATGGCAT AAGGTCTAAG GTACGGACTA
ATTTGTTTGG ACCCAGTGAA GTTACTTACT CTGGCTACAC TTGTTATACT
GGAATTGCAG ATTTTGTTCC TGCTGATATT GATAGAGTTG GGTACCGAGT
CTTTTTGGGC CACAAACAGT ACTTTGTTTC TTCAGATGTG GGGGGAGGCA
AGATGCAGTG GTATGCATTT TACAATGAAC CAGCTGGTGG TGTGGATGCT
CCAAATGGTA AAAAGGAAAG ATTGCTTAAA ATATTTGGGG GATGGTGTGA
CAATGTCATA GACCTATTAG TTGCCACAGA TGAAGATGCA ATTCTTCGTC
GTGACATCTA TGATAGACCA CCAACTTTTA GTTGGGGAAG AGGTCATGTT
ACATTGCTTG GGGACTCTGT CCATGCTATG CAGCCTAATT TGGGTCAAGG
GGGATGCATG GCCATAGAGG ATAGCTATCA ACTAGCACTG GAACTTGACA
AAGCATGTAG TCGAAGTGCC GAGTCAGGAA GCCCTGTGGA TATCATCTCA
TCTTTAAGGA GCTATGAAAG TGCTAGAAAA CTTCGAGTTG GAGTCATCCA
TGGACTGGCT AGAATGGCTG CAATCATGGC ATCTACTTAC AAAGCTTATC
TTGGCGTCGG ACTTGGTCCA CTATCATTTT TGACGCAGTA TAGAATACCA
CATCCTGGAA GAGTTGGTGG AAGAGTATTT ATTGACTTGG GAATGCCTCT
GATGTTAAGT TGGGTTCTAG GAGGCAATGG GGACAAGCTT GAAGGCAGAA
TAAAACATTG CAGGCTATCT GAGAAAGCAA ATGACCAATT GAGA

## Patentansprüche

1. Verfahren zur Erhöhung des Zeaxanthin- und/oder Gesamt-Carotinoid-Gehalts in transgenen Pflanzenzellen, Pflanzen und anderen Wirtszellen/-organismen, wie Algen, **dadurch gekennzeichnet, daß** die endogene Zeaxanthin-Epoxidaseaktivität inhibiert wird.

2. Verfahren nach Anspruch 1, wobei die Inhibierung durch Co-Suppression oder Antisense-Inhibierung erfolgt.

3. Verfahren nach Anspruch 1 oder 2, umfassend die folgenden Schritte:
a) Herstellen eines rekombinanten Nukleinsäuremoleküls, das folgende Elemente umfaßt:
- regulatorische Sequenzen eines in Pflanzenzellen aktiven Promotors;
- in operativer Verknüpfung mit den regulatorischen Sequenzen eine DNA-Sequenz, die Teil einer für eine Zeaxanthin-Epoxidase kodierenden Sequenz ist, wobei diese Sequenz auch ein genomischer Klon sein kann und die DNA-Sequenz in senseoder antisense-Orientierung vorliegt;
- optional in operativer Verknüpfung mit der DNA-Sequenz regulatorische Sequenzen, die als Transkriptions-, Terminations- und/oder Polyadenylierungssequenzen in Pflanzenzellen dienen können;
b) Übertragen des Nukleinsäuremoleküls aus a) auf pflanzliche Zellen; und
c) gegebenenfalls Regenerieren von Pflanzen aus den transformierten Pflanzenzellen.

4. Verfahren nach Anspruch 3, worin die DNA-Sequenz, die Teil einer für eine Zeaxanthin-Epoxidase kodierenden Sequenz ist, aus Kartoffel stammt.

5. Verfahren nach Anspruch 3 oder 4, worin die DNA-Sequenz die in SEQ ID No. 1 angegebene Sequenz ist.

6. Verfahren nach einem der Ansprüche 3 bis 5, worin der in Pflanzenzellen aktive Promotor ein Speicherorgan-spezifischer Promotor ist.

7. Verfahren nach einem der vorangehenden Ansprüche, worin die transgenen Pflanzenzellen oder Pflanzen Kartoffelzellen bzw. Kartoffelpflanzen sind.

8. Transgene Pflanzenzellen oder Pflanzen mit einem gegenüber Wildtypzellen bzw. Wildtyppflanzen erhöhten Zeaxanthin- und/oder Gesamt-Carotinoid-Gehalt, hergestellt nach einem Verfahren gemäß einem der Ansprüche 1 bis 7, sowie Teile dieser Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanzen.

9. Transgene Pflanzenzellen oder Pflanzen nach Anspruch 8, die einen gegenüber den entsprechenden Wildtypzellen bzw. Wildtyppflanzen mindestens 4-fach, bevorzugt mindestens 10-, 20-, 50-fach und besonders bevorzugt mindestens 70-, 100-, 120- und 130-fach erhöhten Zeaxanthin-Gehalt aufweisen.

10. Transgene Pflanzenzellen oder Pflanzen nach Anspruch 9, die einen gegenüber Wildtypzellen bzw. Wildtyppflanzen 2-fach, bevorzugt 3-, 4-fach und besonders bevorzugt 5-fach erhöhten Gesamt-Carotinoid-Gehalt aufweisen.

11. Transgene Pflanzenteile nach Anspruch 8, wobei es sich um transgene Kartoffelknollen handelt, die einen Zeaxanthin-Gehalt von mindestens 10µg/g Trockengewicht, bevorzugt von mindestens 20 µg/g Trockengewicht und besonders bevorzugt von mindestens 40 µg/g Trockengewicht aufweisen.

12. Transgene Pflanzenteile nach Anspruch 8, wobei es sich um transgene Kartoffelknollen handelt, die einen gegenüber Wildtypknollen 3fach, vorzugsweise 4fach und besonders bevorzugt 5 fach erhöhten Gesamt-Carotinoid-Gehalt aufweisen.

13. Verwendung einer DNA-Sequenz, die ein Protein mit der enzymatischen Aktivität einer Zeaxanthin-Epoxidase kodiert, in Form eines Antisense- oder eines Co-Suppressionskonstrukts zur Erhöhung des Zeaxanthin-Gehalts in transgenen Pflanzen, insbesondere in Kartoffelpflanzen, in transgenen Pflanzenzellen und anderen Wirtszellen/-organismen wie Algen.

14. Verwendung einer DNA-Sequenz, die ein Protein mit der enzymatischen Aktivität einer Zeaxanthin-Epoxidase kodiert, in Form eines Antisense- oder eines Co-Suppressionskonstrukts zur Erhöhung des Gesamt-Carotinoid-Gehalts in transgenen Pflanzen, insbesondere in Kartoffelpflanzen, in transgenen Pflanzenzellen und anderen Wirtszellen/-organismen wie Algen.

15. Verwendung nach Anspruch 13 oder 14, wobei die DNA-Sequenz aus Kartoffel stammt.

16. Isolierte DNA-Sequenz, die für ein Protein mit der enzymatischen Aktivität einer Zeaxanthin-Epoxidase kodiert und aus Kartoffel stammt.

17. Isolierte DNA-Sequenz nach Anspruch 16, wobei die DNA-Sequenz der in SEQ ID No. 1 angegebenen DNA-Sequenz entspricht.

18. Verfahren nach einem der Ansprüche 1 bis 7, weiter umfassend die Gewinnung des Zeaxanthins bzw. Gesamt-Carotinoids aus den transgenen Pflanzenzellen, Pflanzen und anderen Wirtszellen/-organismen, wie Algen.

19. Verwendung der nach dem Verfahren nach einem der Ansprüche 1 bis 7 hergestellten transgenen Pflanzen, Pflanzenzellen und anderen Wirtszellen/-organismen, wie Algen, mit erhöhtem Zeaxanthin- und/oder Gesamt-Carotinoid-Gehalt als Produktionsstätte für Zeaxanthin bzw. Gesamt-Carotinoid.

20. Verwendung des nach dem Verfahren nach Anspruch 18 gewonnenen Zeaxanthin bzw. Gesamt-Carotinoid zur Nahrungsergänzung.

21. Verfahren nach einem der Ansprüche 1 bis 7, wobei in die transgenen Pflanzenzellen, Pflanzen und anderen Wirtszellen/-organismen, wie Algen, zusätzlich mindestens ein Gen aus dem Precursor-Carotinoid-Stoffwechsel eingeführt und exprimiert wird.

22. Verfahren nach einem der Ansprüche 1 bis 7, wobei in die transgenen Pflanzenzellen, Pflanzen und anderen Wirtszellen/-organismen, wie Algen, zusätzlich mindestens ein Gen aus dem der Zeaxanthin-Epoxidase nachgelagerten Stoffwechsel eingeführt und exprimiert wird.
